# FASCICULE DE BREVET EUROPEEN

(11) **EP 2 988 766 B1**
(45) Date de publication et mention de la délivrance du brevet: **07.06.2017**
(21) Numéro de dépôt: 14718621.7
(22) Date de dépôt: 22.04.2014
(51) Int. Cl.: A61K 38/16, A61K 9/08, C07K 14/195, A61P 17/00, A61P 27/02, A61K 45/06

(54) **COMPOSITIONS TOPIQUES COMPRENANT DES VARIANTES DE PLI-OB**
TOPISCHE ZUSAMMENSETZUNGEN MIT OB-FALTUNGSVARIANTEN
TOPICAL COMPOSITIONS COMPRISING OB-FOLD VARIANTS

(30) Priorité: 22.04.2013 FR 1353662
(43) Date de publication de la demande: 02.03.2016
(73) Titulaire: AFFILOGIC, 44200 Nantes (FR)
(72) Inventeur: KITTEN, Olivier, 44200 Nantes (FR)
(74) Mandataire: Flesselles, Bruno F.G.
(86) Numéro de dépôt international: PCT/EP2014/058139
(87) Numéro de publication internationale: WO 2014/173899

(56) Documents cités:
- WO-A1-2012/150314
- WO-A2-2008/068637
- ARCUS VICKERY: "OB-fold domains: a snapshot of the evolution of sequence, structure and function", CURRENT OPINION IN STRUCTURAL BIOLOGY, ELSEVIER LTD, GB, vol. 12, no. 6, 1 décembre 2002 (2002-12-01), pages 794-801, XP002432186, ISSN: 0959-440X, DOI: 10.1016/S0959-440X(02)00392-5

## Description

L'invention se rapporte au domaine de la préparation de compositions topiques pour un usage cosmétique ou thérapeutique, contenant des principes actifs se liant à des cibles d'intérêt.

Il existe un certain nombre de maladies dermatologiques, telles que les eczémas, psoriasis, lichen plan et dermatoses bulleuses auto-immunes, ou des cancers de la peau (mélanome). Afin de traiter ces maladies, il est souhaitable de pouvoir disposer de compositions topiques, c'est-à-dire s'appliquant directement sur les lésions de la peau (ou du cuir chevelu) et agissant localement à l'endroit où il est appliqué.

Par ailleurs, de telles compositions topiques sont souhaitables pour le traitement de maladies oculaires, qu'elles soient liées à des dérèglements métaboliques (notamment dus à l'âge) ou à des infections oculaires (conjonctivites sévères, kératites et ulcères cornéens).

Quelle que soit la maladie, il existe généralement une cible d'intérêt thérapeutique sur laquelle les médicaments sont actifs. Ce peut être un récepteur cellulaire, ou une protéine de surface d'un microorganisme. D'une façon générale, il apparaît souhaitable de disposer de compositions permettant l'application de principes actifs agissant sur les cibles d'intérêt thérapeutiques directement sur le lieu d'action, en évitant le plus que possible le passage par la voie systémique. Toutefois, les anticorps ne pénètrent pas facilement la barrière de la peau ou de la cornée.

La demande WO 2007/139397 décrit l'utilisation de banques de protéines à pli-OB dans lesquelles on modifie le domaine OB par l'introduction de mutations dans ce domaine de liaison de la protéine à son ligand naturel.

La demande WO 2008/068637 décrit l'utilisation d'une banque basée sur la protéine Sac7d pour obtenir des ligands présentant une affinité pour des cibles d'intérêt. La méthode décrite dans WO 2008/068637 comprend la génération de banques combinatoires contenant une pluralité de molécules d'ADN ayant toutes la même séquence, sauf la présence de certaines mutations aléatoires menant à la production de variants d'une protéine sauvage, présentant des mutations à certains acides aminés du site de liaison de cette protéine à pli-OB sauvage. En particulier, dans le cadre de WO 2008/068637, la protéine à pli-OB sauvage est une protéine Sac7d, dans laquelle on introduit des mutations pour générer une variabilité, notamment aux acides aminés choisis parmi K7, Y8, K9, K21, K22, W24, V26, M29, S31, T33, T40, R42, A44, S46, ou sur d'autres acides aminés, comme V26, G27, K28, M29, S31, R42, A44, S46, E47 et K48. Ces acides aminés sont basés sur la séquence de Sac7d, telle que représentée par SEQ ID N° 1.

La demande de brevet WO 2012/150314 présente la portabilité de mutations d'une protéine de la famille Sac7d vers une autre protéine de la même famille. Cette portabilité revient à créer un mutant d'une autre protéine de la famille Sac7d à partir d'un mutant d'une protéine de ladite famille, que l'on a pu obtenir notamment en mettant en oeuvre le procédé de WO 2008/068637.

La famille Sac7d est définie comme se rapportant à La famille Sac7d correspond à une famille de protéines de 7 kDa liant l'ADN isolées de bactéries extrémophiles. Ces protéines et cette famille sont notamment décrites dans WO 2008/068637. Ainsi, dans le cadre de la présente invention, une protéine appartient à la famille Sac7d lorsqu'elle présente une séquence correspondant à la séquence SEQ ID N° 8. Cette famille comprend notamment les protéines Sac7d or Sac7e issues de *Sulfolobus acidocaldarius*, Sso7d issue de *Sulfolobus solfataricus*, DBP 7 issue de *Sulfolobus tokodaii*, Ssh7b issue de *Sulfolobus shibatae*, Ssh7a issue de *Sulfolobus shibatae*, et p7ss issue de *Sulfolobus solfataricus*.

Les protéines à pli-OB sont connues dans l'art. Elles sont notamment décrites dans les documents cités plus haut, ainsi que dans Arcus (Curr Opin Struct Biol. 2002 Dec;12(6):794-801). Le pli-OB se présente sous la forme d'un cylindre présentant cinq feuillets beta (β). La plupart des protéines à pli-OB utilisent le même interface de liaison de leur ligand naturel, qui peut être un oligosaccharide, un oligonucléotide, une protéine, un ion métallique ou un substrat catalytique. Cette interface de liaison comprend principalement les résidus situés dans les feuillets beta. Certains résidus situés dans les boucles peuvent aussi être impliqués dans la liaison d'une protéine à pli-OB avec son ligand naturel. Ainsi, les demandes WO 2007/139397 et WO 2008/068637 et le document Arcus (2002, *op*. *cit.*) décrivent les domaines de liaison des protéines à pli-OB avec leur ligand naturel. Ainsi, le document WO 2008/068637 décrit précisément la façon d'identifier le domaine de liaison d'une protéine à pli-OB.

En superposant plusieurs séquences et structures 3D de protéines présentant des domaines à pli-OB en utilisant les sites Web WU-Blast2 (http://www.ebi.ac.uk/blast2/index.html) (Lopez et al., 2003, Nucleic Acids Res 31, 3795-3798), T-COFFEE (http://www.ch.embnet.org/software/TCoffee.html) (Notredame et al., 2000, J Mol Biol 302, 205-217) et DALI lite (http://www.ebi.ac.uk/DaliLite/) (Holm et Park, 2000, Bioinformatics 16, 566-567) on peut identifier les positions des domaines de liaison et notamment les acides aminés qui peuvent être modifiés. En prenant comme référence la séquence de Sac7d (SEQ ID N °1), il s'agit des résidus V2, K3, K5, K7, Y8, K9, G10, E14, T17, K21, K22, W24, V26 , G27, K28, M29, S31, T33, D36, N37, G38, K39, T40, R42, A44, S46, E47, K48, D49, A50 et P51. Toujours avec cette séquence Sac7d en tant que référence, les résidus qui peuvent être supprimés sont: A59, R60, A61 et E64.

L'identification des domaines de liaison d'autres protéines à pli-OB peut être effectuée, ainsi que décrit dans WO 2008/068637. Cette demande décrit que l'on peut effectuer un superposition de structures 3D de protéines ou domaines pli-OB (10 domaines ont été utilisés dans cette demande, y compris Sac7d), en utilisant le site Web DALI (http://www.ebi.ac.uk/dali/Interactive.html) (Holm et Sander, 1998, Nucleic Acids Res 26, 316-319). Ainsi, il est aisé d'identifier, pour toute protéine (ou tout domaine) à pli-OB, les acides aminés impliqués dans le site de liaison et correspondant aux acides aminés de Sac7d mentionnés ci-dessus.

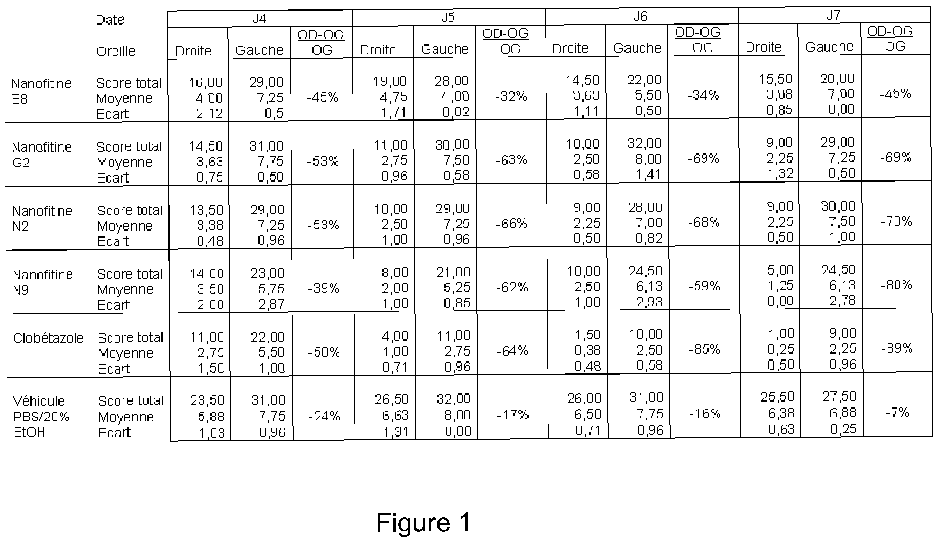

L'enseignement de WO 2008/068637 indique également que l'on peut éventuellement insérer des acides aminés dans les boucles des protéines à pli-OB, notamment les protéines de la famille Sac7d, en particulier des insertions de 1 à 15 résidus d'acides aminés peuvent être effectuées dans la boucle 3 (telle que définie dans les figures 1b et 2 de WO 2008/068637), par exemple dans la région des résidus 25 à 30 de Sac7d, de préférence entre les résidus 27 et 28, des insertions de 1 à 15 résidus d'acides aminés peuvent être effectuées dans la boucle 4 (telle que définie dans les figures 1b et 2 de WO 2008/068637), par exemple dans la région des résidus 35-40 de Sac7d, de préférence entre les résidus 37 et 38, et des insertions de 1 à 20 résidus peuvent être effectuée dans la boucle 1 (telle que définie dans les figures 1 b et 2 de WO 2008/068637), par exemple dans la région des résidus 7 à 12 de Sac7d, de préférence entre les résidus 9 et 10.

Par extension, dans le cadre de la présente demande, le terme « protéine à pli-OB » comprend également les domaines présentant un pli-OB que l'on peut isoler de protéines plus complexes. Ces domaines à pli-OB sont notamment décrits plus en détail dans les demandes WO 2007/139397 et WO 2008/068637.

L'intérêt de la méthode décrite dans WO 2008/068637 est qu'elle permet d'obtenir des variants de protéines à pli-OB par criblage de banques combinatoires contenant ou exprimant une pluralité de variants dans lesquels un certain nombre d'acides aminés ont été « randomisés », c'est-à-dire remplacés par un acide aminé aléatoire. Le criblage de ces banques permet d'identifier des variants de ces protéines qui se lient de façon spécifique, généralement avec une forte affinité (la demande WO 2008/068637 décrit en effet des affinités de l'ordre du nanomolaire), avec une cible d'intérêt, différente du ligand naturel de la protéine sauvage à partir de laquelle la banque combinatoire a été générée.

Les inventeurs ont montré que les protéines à pli-OB sont capables de passer la barrière oculaire ou de la peau et peuvent donc être utilisées dans des compositions topiques. On utilise de préférence un variant d'une protéine à pli-OB, qui se lie à une cible d'intérêt, ladite cible d'intérêt étant impliquée dans une pathologie, notamment ophtalmologique ou dermatologique.

La présente invention se rapporte ainsi à une composition topique comprenant une protéine à pli-OB ou un variant d'une protéine sauvage à pli-OB, ledit variant présentant entre 5 et 32 résidus mutés dans l'interface de liaison de ladite protéine sauvage à pli-OB à son ligand naturel. Dans un mode de réalisation particulier, ledit variant se lie de façon spécifique à une cible d'intérêt différente dudit ligand naturel. Ce variant a donc généralement été identifié par la mise en oeuvre d'une méthode telle que décrite dans WO 2008/068637 ou WO 2007/139397. De fait, la mise en oeuvre des méthodes décrites dans ces deux demandes de brevet permet généralement d'identifier des variants de toute protéine à pli-OB se liant à toute cible d'intérêt. Cette composition contient également généralement un excipient à usage topique, pharmaceutiquement ou cosmétiquement acceptable

Ainsi que vu plus haut, ladite protéine sauvage à pli-OB englobe aussi les domaines à pli-OB. D'une façon préférée, le variant mis en oeuvre dans le cadre de la présente invention contient au maximum 150 acides aminés, de façon plus préférée au maximum 100 acides aminés. Dans un mode de réalisation particulier, il contient au maximum 70 acides aminés.

Le nombre de résidus mutés dans ledit variant (par rapport à la protéine sauvage) est compris entre 5 et 32. Dans d'autres modes de réalisation, ces variants présentent de préférence au moins 5, de façon plus préférée au moins 8, de façon encore plus préférée au moins 10, mais généralement moins de 32, de façon plus préférée moins de 24, de façon encore plus préférée moins de 20 ou moins de 15 acides aminés substitués par rapport à la protéine (ou au domaine) à pli-OB sauvage. On préfère lorsque 8, 9, 10, 11, 12 ou 13 acides aminés sont mutés par rapport à la protéine sauvage. Ces acides aminés mutés sont localisés dans le site de liaison de la protéine à pli-OB avec son ligand naturel. Ils sont généralement répartis sur l'ensemble de ce domaine de liaison. Étant donné la structure de ce domaine de liaison, certains résidus mutés se trouvent dans un (généralement plusieurs, notamment deux ou trois) feuillet beta.

Dans un mode de réalisation particulier, ces variants peuvent également comprendre des insertions d'acides aminés dans des boucles reliant les feuillets beta du pli-OB. Ainsi, on peut introduire entre 1 et 15 acides aminés dans la boucle 1 et/ou dans la boucle 4 et/ou dans la boucle 3 (les boucles étant numérotées de la même façon que dans WO 2008/068637.

Dans un mode de réalisation particulier, ladite protéine sauvage à pli-OB est choisie parmi Sac7d or Sac7e issues de *Sulfolobus acidocaldarius*, Sso7d issue de *Sulfolobus solfataricus*, DBP 7 issue de *Sulfolobus tokodaii*, Ssh7b issue de *Sulfolobus shibatae*, Ssh7a issue de *Sulfolobus shibatae*, et p7ss issue de *Sulfolobus solfataricus*.

Il s'agit donc de la protéine à laquelle est comparée le variant mis en oeuvre dans la composition topique de la présente invention.

Les différentes séquences des protéines Sac7d, Sso7d, Sac7e, Ssh7b, SSh7a, DBP7 et Sis7 sont représentées par SEQ ID N° 1 à SEQ ID N° 7 respectivement.

Un variant d'une protéine de cette famille Sac7d est appelée une nanofitine. La mise en oeuvre de l'invention se fait ainsi préférentiellement sur des variants des protéines représentées par SEQ ID N° 1 à SEQ ID N° 7, en particulier sur des variants de Sac7d.

La concentration du variant dans la composition selon la présente invention est généralement supérieure à 10 ng/ml et inférieure à 600 mg/ml. En effet, dans la cas d'une application oculaire, la concentration de protéine n'a pas besoin d'être très importante. Elle est donc généralement inférieure à 10 mg/ml, de préférence inférieure 1 mg/ml, de préférence inférieure à 500 ng/ml, ou à 250 ng / ml, voire inférieure à 100 ng/ml. De même, si la concentration peut être aussi faible que 10 ng / ml, elle est de préférence supérieure à 50 ng / ml, voire supérieure à 100 ng / ml.

Dans le cadre d'une utilisation par voie topique par application sur la peau (ou le cuir chevelu), la concentration du produit peut généralement être plus importante. Ainsi, la concentration sera généralement supérieure à 10 mg / ml, de préférence supérieure à 50 mg / ml, de façon plus préférée supérieure à 100 mg / ml, de façon encore plus préférée supérieure à 200 mg / ml.

Le variant présent dans la composition selon l'invention se lie de façon spécifique à une cible d'intérêt. De fait, il a été sélectionné, par une méthode telle que décrite dans WO 2008/068637 ou WO 2007/139397 pour sa spécificité de liaison avec cette cible d'intérêt. Par ailleurs, les méthodes décrites dans ces demandes de brevets permettent d'obtenir des affinités de l'ordre du micromolaire (WO 2007/139397) ou du nanomolaire (WO 2008/068637).

Les cibles d'intérêt sont choisies en fonction de la maladie que l'on souhaite traiter. On peut ainsi citer tout antigène, anticorps, protéine cellulaire, protéine circulante, peptides. On peut également cibler un principe actif de médicament, ou un acide nucléique particulier. On envisage en particulier que la cible d'intérêt soit une interleukine, une cytokine, un récepteur aux cytokines ou aux interleukines, une protéine codée par un oncogène, une protéine de surface d'un microorganisme, ou un lipopolysaccharide de microorganisme.

Dans un mode de réalisation particulier, la composition topique selon l'invention est destinée à être appliquée sur la peau.

Dans un autre mode de réalisation, cette composition topique est destinée à être appliquée au niveau oculaire.

La forme de la composition topique selon l'invention est classique, et dépend notamment du lieu d'administration (peau ou oeil).

Ainsi, cette composition peut se présenter sous une forme liquide (notamment un collyre pour une application oculaire), pâteuse, ou solide. Elle peut se présenter sous la forme d'une crème d'une pommade, d'un onguent d'une poudre, d'un gel, d'une émulsion (huile dans eau ou eau dans huile), d'une mousse. Des formulations à base aqueuse contenant ou non des solvants organiques (tel que éthanol) sont particulièrement adaptées pour ces compositions.

Elle peut aussi se présenter sous la forme de tampon imbibé, de lingette, de spray, d'un aérosol, de lotion, de stick, de shampooing.

La composition selon l'invention peut également se présenter sous forme de suspension de microsphères ou nanosphères ou de vésicules lipidiques ou polymériques ou de patchs polymériques et d'hydrogels permettant une libération contrôlée des principes actifs. Cette composition pour application topique peut se présenter sous forme anhydre, sous forme aqueuse ou sous la forme d'une émulsion.

Dans un mode de réalisation particulier, la composition topique selon l'invention se présente sous la forme d'un « patch » (système transdermal). De tels systèmes peuvent notamment permettre une libération contrôlée du principe actif.

Un tel système comprend généralement une feuille protectrice (couche de support) imperméable à la substance d'intérêt (le variant de la protéine à pli-OB dans le cadre de la présente invention), un réservoir contenant la substance d'intérêt, un élément contrôlant la livraison de la substance d'intérêt sur la peau, à travers une couche adhésive (sensible à la pression) permettant le maintien du dispositif sur la peau, et une couche-protectrice détachable avant application sur la peau. Il est possible que le même élément accomplisse plusieurs fonctions (par exemple réservoir, contrôle de libération et adhésion). De tels dispositifs sont connus dans l'état de la technique.

Les variants des protéines à pli-OB utilisés dans la composition selon l'invention peuvent être produits par synthèse chimique en phase solide ou par recombinaison génétique. La synthèse chimique peut être réalisée par exemple avec un synthétiseur automatique de peptide du type Applied Biosystems, mod. 433A., ou en chimie Fmoc qui utilise le groupement fluoremylméthyloxycarbonyle pour la protection temporaire de la fonction a-aminique des acides aminés.

Il est toutefois préféré de produire les variant utilisables dans le cadre de l'invention par génie génétique, en particulier en intégrant une séquence d'acide nucléique codant pour ledit polypeptide dans un vecteur d'expression. Ce vecteur d'expression est alors introduit dans une cellule hôte (les bactéries telles *Escherichia coli* sont particulièrement adaptées), qui est cultivée dans des conditions de culture permettant la synthèse du polypeptide (on peut notamment utiliser des promoteurs inductibles en amont du polypeptide dans le vecteur d'expression. Le polypeptide synthétisé est alors récupéré. On peut alors greffer tout type de molécules en N- ou C-terminal du polypeptide.

L'homme du métier connaît les méthodes de production de polypeptides, qui sont notamment décrites dans l'ouvrage de Sambrook, Fritsch et Maniatis, MOLECULAR CLONING, A LABORATORY MANUAL, 2nde édition.

La composition selon l'invention peut contenir des variants de la protéine à pli-OB seuls ou avec d'autres principes actifs. Ainsi, la composition peut aussi contenir au moins un agent choisi parmi les agents antibactériens, antiparasitaires, antifongiques, anti-inflammatoires, antiprurigineux, anesthésiques, antiviraux, kératolytiques, anti-radicaux libres, antiséborrhéiques, antipelliculaires, antiacnéiques et les agents modulant la différenciation et/ou la prolifération et/ou la pigmentation cutanée.

Cette composition peut également contenir un agent régulateur de pH, notamment pour permettre la régulation du pH autour de pH = 7.

Les variants de la protéine à pli-OB peuvent aussi être fusionnés avec une protéine active, notamment afin d'augmenter leur temps de demi-vie.

La composition selon l'invention peut aussi être utilisée dans une méthode de diagnostic *in vivo*. Ainsi, dans un mode de réalisation particulier, le variant de la protéine à pli-OB est couplé à un agent de détection ou de contraste. Ceci permet notamment un suivi de cet agent de contraste et de déterminer les organes sur lesquels le variant de la protéine à pli-OB se lie (ce qui indique la présence de la cible d'intérêt).

L'invention se rapporte aussi à un procédé de préparation d'une composition topique, comprenant l'étape de mélanger un variant d'une protéine sauvage à pli-OB à un excipient à usage topique, pharmaceutiquement ou cosmétiquement acceptable.

Dans un mode de réalisation particulier, ladite composition est destinée à être appliquée sur la peau. L'excipient permet, de préférence, donc la réalisation d'une composition qui peut être étalée sur la peau, telle qu'une pommade, un onguent ou une lotion.

Dans un autre mode de réalisation, ladite composition est destinée à être appliquée sur l'oeil, et les excipients sont choisis en conséquence. On obtient ainsi notamment des collyres, des gouttes d'oeil.

L'invention se rapporte également à une composition topique, selon l'invention, contenant un variant d'une protéine sauvage à pli-OB, ledit variant présentant entre 5 et 32 résidus mutés dans l'interface de liaison de ladite protéine sauvage à pli-OB à son ligand naturel, en tant que médicament. Dans ce cas particulier ledit variant se liant de façon spécifique à une cible d'intérêt, qui est une cible thérapeutique.

L'invention se rapporte également à une composition topique selon l'invention pour le traitement d'une maladie ou d'un désordre dermatologique ou ophtalmologique. Dans ce mode de réalisation, le variant de la protéine à pli-OB utilisé cible (se lie à) un facteur étiologique de la maladie visée ou une cible thérapeutique liée à cette maladie.

Dans ce mode de réalisation, on sélectionne donc un variant de la protéine à pli-OB, notamment par les méthodes de criblage décrites dans WO 2008/068637, qui se lie à une cible thérapeutique (effecteur ou facteur étiologique) impliquée dans la maladie ou le désordre que l'on souhaite traiter, et on formule ce variant dans une composition pour application topique.

L'invention se rapporte également à une composition cosmétique comprenant une composition topique selon l'invention et à l'utilisation cosmétique d'une composition topique selon l'invention.

L'invention se rapporte également à une méthode de traitement d'une maladie ou d'un désordre dermatologique ou ophtalmologique, caractérisée en ce que l'on applique localement (sur la peau ou au niveau oculaire selon le besoin) une composition topique selon l'invention, contenant une protéine à pli-OB ciblant, de préférence, un facteur étiologique ou une cible thérapeutique caractéristique de ladite maladie ou dudit désordre.

### Exemples

Dans l'ensemble des exemples, on utilise des nanofitines, telles que définies ci-dessus, c'est-à-dire des variants de Sac7d, obtenus par criblage d'une banque combinatoire contre une cible d'intérêt, selon une méthode similaire à la méthode décrite dans WO 2008/068637. L'objectif de ces exemples étant de démontrer que les protéines à pli-OB, et notamment les nanofitines sont capables de passer la barrière de la peau ou de pénétrer dans l'oeil, sans que ceci ne soit dépendant de la séquence de la protéine, les séquences des nanofitines et les cibles d'intérêt ne sont pas précisées.

### Exemple 1 : test de pénétration de nanofitines à travers un modèle de peau humaine

Le test utilisé est le test de diffusion des cellules de Franz.

Les cellules de Franz servent déterminer, *ex vivo*, la pénétration cutanée de substances exogènes, et de mesurer effectivement la vitesse de passage à travers les différentes couches de la barrière cutanée des molécules constituant le principe actif déposé à la surface de la peau.

Une solution contenant la molécule à étudier est déposée dans un compartiment « donneur » du dispositif. La molécule diffuse ensuite à travers une membrane (la peau en l'occurrence) et est collectée dans un compartiment « récepteur ».

En pratique, après avoir retiré la couche hypodermique (tissu adipeux), le fragment de peau est déposé sur une cellule de diffusion, appelée cellule de Franz. La face profonde du derme est en contact avec une solution réceptrice qui est censée remplacer le liquide interstitiel de la peau. Une solution contenant la molécule étudiée est déposée au-dessus de la peau. A intervalle de temps régulier, la solution réceptrice est prélevée afin de doser la molécule qui a traversé la peau dégraissée et une nouvelle solution lui est alors substituée.

Le dosage de substances présentes dans la phase réceptrice prélevée permet de déterminer soit la quantité de molécules ayant traversée la peau, soit le flux (en nanogramme/cm²/heure).

On a testé des nanofitines issues de criblages d'une banque de variants de Sac7d pour leur affinité contre trois cibles d'intérêt différentes, impliquées dans des maladies cutanées ou ophtalmologiques. Ces nanofitines ont été produites dans un système bactérien et se lient donc à des ligands différents.

On a utilisé 6 fragments de peau humaine (épaisseur 1 mm), décongelés issus du même donneur.

Le compartiment récepteur était rempli de PBS (tampon phosphate salin, pH 7,4 ; 5,8 ml). La membrane a été placée sur le milieu récepteur.

On a déposé 100 µl de chaque formulation contenant 5mg/ml de nanofitine sur la membrane. La surface d'échange était de 2 cm².

La température a été maintenue à 35°C pendant toute l'expérience du côté récepteur, correspondant à une température de 32° C à la surface externe de la peau humaine.

À chaque période d'échantillonnage suivante : 0 h et 24h, on a prélevé 500 µl de liquide du côté récepteur que l'on a remplacé par du PBS.

Du côté donneur, à t=24h, la peau a été rincée avec 5 ml de PBS.

À la fin de la période d'échantillonnage, les 2cm² de peau ont été retirés du dispositif de cellules de Franz et placés dans un bain à ultra-son dans 1 ml de PBS.

On a mesuré sur chacun des échantillons prélevés la concentration en nanofitines par la technologie de BioLayer Interferometry qui permet de mesurer l'interaction entre deux protéines. En l'occurrence, la teneur en nanofitines est mesurée par la reconnaissance d'un tag poly-Histidine qui leur est préalablement couplé.

Les résultats sont les suivants :

Le pourcentage de nanofitines retrouvé du côté récepteur s'échelonne, selon la nanofitine, entre 8% et 17%. Le standard de perméabilité de la peau utilisé est la caféine, qui dans le contexte expérimental présente une perméabilité de l'ordre de 10%.

Ces résultats montrent que les nanofitines traversent effectivement la peau humaine, et que ce résultat n'est pas dépendant de la cible contre laquelle la nanofitine a été générée et se lie.

### Exemple 2 : test de pénétration de nanofitines à travers la cornée de lapin

On a utilisé trois lapins mâles (Fauve de Bourgogne).

Le principe est le même que pour la perméabilité de la peau.

2 chambres de réaction ont été utilisées pour l'étude de la perméabilité de chaque formulation (NFI, NF2 et NF3).

3 ml de formulation contenant chaque nanofitine ont été placés du côté donneur des chambres de réaction et 3ml de PBS ont été placés du côté récepteur. Des tissus cornéens fraîchement prélevés ont été placés entre les deux demi-chambres.

La température a été maintenue à 37°C pendant toute l'étude et l'oxygénation a été fournie par une perfusion continue de carbogène (95 % O₂, 5 % CO₂). La surface d'échange était de 0,5cm².

À chaque période d'échantillonnage suivante : 0h ; 2h et 4h, on a prélevé 500 µl de liquide du côté récepteur que l'on a remplacé par du PBS.
- 100 µl de liquide ont été prélevés du côté donneur et n'ont pas été remplacés.

À la fin de la période d'échantillonnage, les 0,5 cm² de cornées ont été retirés de la chambre de réaction et grattés avec un scalpel. 1 mL de PBS a été ajouté aux échantillons qui ont été homogénéisés.

On a mesuré sur chacun des échantillons prélevés la concentration en nanofitines par la technologie de BioLayer Interferometry qui permet de mesurer l'interaction entre deux protéines. En l'occurrence, la teneur en nanofitines est mesurée par la reconnaissance d'un tag poly-Histidine qui leur est préalablement couplé.

Les résultats sont les suivants :

La quantité de nanofitines retrouvée du côté récepteur permet de calculer une perméabilité apparente (cm/s) comprise, selon les nanofitines, entre 5 10⁻⁶ et 2.10⁻⁵. En comparaison avec les standards utilisés dans ce type de protocole expérimental, les nanofitines testées présentent une perméabilité comparable à celle de la norfloxacine (1,1.10⁻⁵ dans ce modèle expérimental), qui est une molécule présentant une bonne pénétration de la cornée et qui est utilisé en ophtalmologie dans un collyre pour le traitement antibactérien local des infections oculaires sévères dues à des germes sensibles à la norfloxacine.

Ces résultats montrent que les nanofitines traversent effectivement la cornée, et que ce résultat n'est pas dépendant de la cible contre laquelle la nanofitine a été criblée.

### Exemple 3 : évaluation de la perméabilité cutanée des nanofitines in vivo

### Objectif :

Afin de vérifier la pénétration cutanée de nanofitines et la conservation de leurs propriétés notamment de neutralisation de la relation récepteur-ligand, des modèles d'inflammation de type psoriasis ont été utilisés et on a mesuré la réduction de l'inflammation (Score PASI) après administration topique de nanofitines anti-inflammatoires directement sur la peau.

Ces nanofitines ciblent deux cytokines différentes impliquées dans l'inflammation psoriasiforme mais avec des localisations différentes, depuis les couches basales du derme jusque dans l'épiderme (Nestle et al., Nature Reviews Immunology 9, 679-691, 2009). Les nanofitines en question neutralisent la liaison des cytokines ciblées avec leur récepteur. Pour inhiber l'action de ces cytokines lors de l'inflammation de type psoriasis, il faut atteindre les couches où elles sont localisées soit par la voie systémique comme le font les anticorps thérapeutiques, soit par la voie cutanée topique. La mesure d'un effet anti-inflammatoire par administration topique de nanofitines témoigne ainsi d'une pénétration des Nanofitines au travers de la peau et d'une efficacité thérapeutique associée.

### Méthode :

L'application locale d'imiquimod (Aldara) sur la face interne des oreilles de souris provoque une inflammation cutanée proche du psoriasis (Van der Fits et al., J Immunol. 2009 May 1;182(9):5836-45, Flutter et al., Eur J Immunol. 2013 Dec;43(12):3138-46). Ce protocole a été appliqué sur des souris Balb/c de 6-8 semaines, sur les deux oreilles.

On a utilisé quatre groupes de souris
- Un groupe témoin, dans lequel les souris n'ont pas reçu d'imiquimod (contrôle négatif)
- Trois groupes expérimentaux, dans lesquels les souris ont reçu de l'imiquimod sur les deux oreilles, ainsi qu'un traitement sur l'oreille droite. Ces trois groupes expérimentaux sont :
   ∘ Un groupe de test de l'efficacité de nanofitines : Quatre nanofitines différentes ont été appliquées sur l'oreille droite des souris (n=4) pour chaque sous-groupe expérimental de ce groupe. Les nanofitines étaient formulées dans du PBS avec éventuellement 20%EtOH.
   ∘ Un groupe contrôle dans lequel les souris ont reçu le véhicule seul (PBS, 20%EtOH) des nanofitines sur l'oreille traitée
   ∘ Un groupe contrôle dans lequel les souris ont un traitement contrôle (clobetazole) de façon topique comme contrôle positif de traitement.

Le protocole a été organisé comme suit :
- Administration quotidienne de l'imiquimod pendant 7 jours consécutifs, 1 adm/jour le matin
- Administration des nanofitines ou des autres contrôles à partir du troisième jour jusqu'au septième jour, 1 adm/jour le soir
- Observation et détermination du score PASI du jour 4 au jour 7, le matin avant administration de l'imiquimod.
- Sacrifice le huitième jour après détermination du score PASI.

La mesure de l'efficacité a consisté en l'observation par deux opérateurs de façon indépendante et en aveugle des paramètres du score PASI pour chaque oreille : épaisseur, desquamation, rougeur sur un grade de 0 (plus faible) à 4 (plus élevé). Un score total est obtenu par l'addition des différents scores pour chaque oreille de chaque animal.

Les quatre nanofitines ciblent des cytokines impliquées dans le phénotype inflammatoire provoqué par l'imiquimod et empêchent celles-ci de se lier à leurs récepteurs respectifs, comme cela a été préalablement vérifié in vitro (BioLayer Interferometry). Elles ont été administrées à 10 mg/kg (à l'exception de la seconde à 20 mg/kg) dans une solution de PBS/20% EtOH ou PBS sans éthanol.

### Résultats :

Pour toutes les nanofitines appliquées, une réduction très significative de l'inflammation sur l'oreille traitée a été constatée en comparaison avec l'oreille non traitée (Figure 1). L'inflammation des oreilles non traitées a été conservée quel que soit le groupe à l'exception de celui ayant reçu le clobétazole.

Le clobétazole a été administré en crème et les souris de ce groupe se sont abondamment léché les oreilles dans ce groupe seulement. On observe également une réduction de l'inflammation sur l'oreille gauche non traitée, dans ce groupe. Un effet systémique après ingestion est donc ainsi très probable avec un effet sur les deux oreilles.

### Conclusion

L'administration topique de nanofitines anti-inflammatoires a permis d'induire une réduction de plus de 30% et pouvant dépasser les 80% des manifestations inflammatoires après 5 jours de traitement.

Ces scores témoignent d'une efficacité très significative *in vivo* des nanofitines utilisées, et démontrent leur capacité à franchir naturellement les barrières cellulaires dans des formulations neutres, tout en conservant une activité neutralisante. Les nanofitines peuvent ainsi être utilisées comme traitements à la fois ciblés et topiques pour le traitement de pathologies dont les cibles ou effecteurs sont sous la peau.

## Revendications

1. Composition topique comprenant un variant d'une protéine sauvage à pli-OB, ledit variant présentant entre 5 et 32 résidus mutés dans l'interface de liaison de ladite protéine sauvage à pli-OB à son ligand naturel.

2. Composition selon la revendication 1, **caractérisée en ce que** ladite protéine sauvage à pli-OB est choisie parmi Sac7d or Sac7e issues de *Sulfolobus acidocaldarius*, Sso7d issue de *Sulfolobus solfataricus*, DBP 7 issue de *Sulfolobus tokodaii*, Ssh7b issue de *Sulfolobus shibatae*, Ssh7a issue de *Sulfolobus shibatae*, et p7ss issue de *Sulfolobus solfataricus*.

3. Composition selon l'une des revendications 1 ou 2, **caractérisée en ce qu'**elle contient entre 10 ng/ml et 600 mg/ml dudit variant.

4. Composition selon l'une des revendications 1 à 3, **caractérisée en ce que** ledit variant se lie à une cible d'intérêt choisie parmi les antigènes, les anticorps, les protéines cellulaires, les protéines circulantes, les peptides, les principes actifs de médicaments, les acides nucléiques, et notamment les interleukines, les cytokines, les récepteurs aux cytokines ou interleukines, les protéines codées par des oncogènes, les protéines de surfaces de microorganismes, et les lipopolysaccharides de microorganismes.

5. Composition selon l'une des revendications 1 à 4, **caractérisée en ce qu'**elle est adaptée à une application sur la peau.

6. Composition selon l'une des revendications 1 à 4, **caractérisée en ce qu'**elle est adaptée à une application au niveau oculaire.

7. Composition selon l'une des revendications 1 à 6, **caractérisée en ce qu'**elle se présente sous la forme d'une solution aqueuse, huileuse ou hydroalcoolique, d'une émulsion, d'une microémulsion, d'un gel aqueux, d'un gel anhydre, d'un sérum, d'une dispersion de vésicules, d'une lotion, d'un gel, d'une crème, d'une mousse, d'un aérosol ou d'un spray.

8. Composition selon l'une des revendications 1 à 7, **caractérisée en ce qu'**elle contient en outre au moins un agent choisi parmi les agents antibactériens, antiparasitaires, antifongiques, anti-inflammatoires, antiprurigineux, anesthésiques, antiviraux, kératolytiques, anti-radicaux libres, antiséborrhéiques, antipelliculaires, antiacnéiques et les agents modulant la différenciation et/ou la prolifération et/ou la pigmentation cutanée.

9. Composition topique selon l'une des revendications 1 à 8, pour son utilisation dans le traitement d'un désordre ou d'une maladie dermatologique ou ophtalmologique.

10. Procédé de préparation d'une composition topique selon l'une des revendications 1 à 9, comprenant l'étape de mélanger un variant d'une protéine sauvage à pli-OB, ledit variant présentant entre 5 et 32 résidus mutés dans l'interface de liaison de ladite protéine sauvage à pli-OB à son ligand naturel à un véhicule pharmaceutiquement ou cosmétiquement acceptable.

## Patentansprüche

1. Topische Zusammensetzung, die eine Variante eines Wildtyp-OB-Faltungs-Proteins umfasst, wobei die Variante zwischen 5 und 32 mutierte Reste in der Bindungsgrenzfläche des Wildtyp-OB-Faltungs-Proteins mit seinem natürlichen Liganden aufweist.

2. Zusammensetzung nach Anspruch 1, **dadurch gekennzeichnet, dass** das Wildtyp-OB-Faltungs-Protein aus von *Sulfolobus acidocaldarius* stammendem Sac7d oder Sac7e, von *Sulfolobus solfataricus* stammendem Sso7d, von *Sulfolobus tokodaii* stammendem DBP 7, von *Sulfolobus shibatae* stammendem Ssh7b, von *Sulfolobus shibatae* stammendem Ssh7a und von *Sulfolobus solfataricus* stammendem p7ss ausgewählt ist.

3. Zusammensetzung nach einem der Ansprüche 1 oder 2, **dadurch gekennzeichnet, dass** sie zwischen 10 ng/ml und 600 mg/ml der Variante enthält.

4. Zusammensetzung nach einem der Ansprüche 1 bis 3, **dadurch gekennzeichnet, dass** die Variante sich an ein Ziel von Interesse bindet, das aus Antigenen, Antikörpern, zellulären Proteinen, zirkulierenden Proteinen, Peptiden, Arzneimittelwirkstoffen, Nukleinsäuren und insbesondere Interleukinen, Cytokinen, Cytokin- oder Interleukinrezeptoren, durch Onkogene kodierten Proteinen, Oberflächenproteinen von Mikroorganismen und Lipopolysacchariden von Mikroorganismen ausgewählt ist.

5. Zusammensetzung nach einem der Ansprüche 1 bis 4, **dadurch gekennzeichnet, dass** sie für eine Anwendung auf die Haut ausgelegt ist.

6. Zusammensetzung nach einem der Ansprüche 1 bis 4, **dadurch gekennzeichnet, dass** sie für eine Anwendung am Auge ausgelegt ist.

7. Zusammensetzung nach einem der Ansprüche 1 bis 6, **dadurch gekennzeichnet, dass** sie in Form einer wässrigen, öligen oder wässrig-alkoholischen Lösung, einer Emulsion, einer Mikroemulsion, eines wässrigen Gels, eines wasserfreien Gels, eines Serums, einer Vesikeldispersion, einer Lotion, eines Gels, einer Creme, eines Schaums, eines Aerosols oder eines Sprays vorliegt.

8. Zusammensetzung nach einem der Ansprüche 1 bis 7, **dadurch gekennzeichnet, dass** sie außerdem mindestens ein Mittel enthält, das aus antibakteriellen Mitteln, antiparasitischen Mitteln, Fungiziden, Entzündungshemmern, Juckreiz stillenden Mitteln, Anästhetika, antiviralen Mitteln, keratolytischen Mitteln, Mitteln gegen freie Radikale, Antiseborrhömitteln, Antischuppenmitteln, Antiaknemitteln und Mitteln, die die Differenzierung und/oder Proliferation und/oder Pigmentierung der Haut modulieren, ausgewählt ist.

9. Topische Zusammensetzung nach einem der Ansprüche 1 bis 8 für die Verwendung bei der Behandlung einer dermatologischen oder ophthalmologischen Störung oder Krankheit.

10. Verfahren zur Herstellung einer topischen Zusammensetzung nach einem der Ansprüche 1 bis 9, umfassend den Schritt des Mischens einer Variante eines Wildtyp-OB-Faltungs-Proteins, wobei die Variante zwischen 5 und 32 mutierte Reste in der Bindungsgrenzfläche des Wildtyp-OB-Faltungs-Proteins mit seinem natürlichen Liganden aufweist, mit einem pharmazeutisch oder kosmetisch annehmbaren Träger.

## Claims

1. A topical composition comprising a variant of a wild-type OB-fold protein, said variant having between 5 and 32 mutated residues in the interface of binding of said wild-type OB-fold protein to its natural ligand.

2. The composition as claimed in claim 1, **characterized in that** said wild-type OB-fold protein is chosen from Sac7d or Sac7e derived from *Sulfolobus acidocaldarius*, Ss07d derived from *Sulfolobus solfataricus*, DBP 7 derived from *Sulfolobus tokodaii*, Ssh7b derived from *Sulfolobus shibatae*, Ssh7a derived from *Sulfolobus shibatae*, and p7ss derived from *Sulfolobus solfataricus*.

3. The composition as claimed in either of claims 1 and 2, **characterized in that** it contains between 10 mg/ml and 600 mg/ml of said variant.

4. The composition as claimed in one of claims 1 to 3, **characterized in that** said variant binds to a target of interest chosen from antigens, antibodies, cell proteins, circulating proteins, peptides, active ingredients of medicaments, nucleic acids, and in particular interleukins, cytokines, cytokine or interleukin receptors, proteins encoded by oncogenes, surface proteins of microorganisms, and microorganism lipopolysaccharides.

5. The composition as claimed in one of claims 1 to 4, **characterized in that** it is adapted to be applied to the skin.

6. The composition as claimed in one of claims 1 to 4, **characterized in that** it is adapted to be applied at the ocular level.

7. The composition as claimed in one of claims 1 to 6, **characterized in that** it is in the form of an aqueous, oily or aqueous-alcoholic solution, an emulsion, a microemulsion, an aqueous gel, an anhydrous gel, a serum, a dispersion of vesicles, a lotion, a gel, a cream, a foam, an aerosol or a spray.

8. The composition as claimed in one of claims 1 to 7, **characterized in that** it also contains at least one agent chosen from antibacterial, antiparasitic, antifungal, anti-inflammatory, antipruritic, anesthetic, antiviral, keratolytic, free-radical-scavenger, antiseborrheic, antidandruff and anti-acne agents and agents for modulating the differentiation and/or proliferation and/or pigmentation of the skin.

9. The topical composition as claimed in one of claims 1 to 8, for use thereof in treating a dermatological or ophthalmological disorder or disease.

10. A process for preparing a topical composition as claimed in one of claims 1 to 9, comprising the step of mixing a variant of a wild-type OB-fold protein, said variant having between 5 and 32 mutated residues in the interface of binding of said wild-type OB-fold protein to its natural ligand, with a pharmaceutically or cosmetically acceptable carrier.


### SEQUENCE LISTING

<110> AFFILOGIC
<120> Compositions topiques
<130> BRV 75 - WO
<150> FR 13/53662
   <151> 2013-04-22
<160> 8
<170> PatentIn version 3.3
<210> 1
   <211> 66
   <212> PRT
   <213> Sulfolobus acidocaldarius
<400> 1
   Met Val Lys Val Lys Phe Lys Tyr Lys Gly Glu Glu Lys Glu Val Asp
   1               5                   10                  15
   Thr Ser Lys Ile Lys Lys Val Trp Arg Val Gly Lys Met Val Ser Phe
               20                  25                  30
   Thr Tyr Asp Asp Asn Gly Lys Thr Gly Arg Gly Ala Val Ser Glu Lys
           35                  40                  45
   Asp Ala Pro Lys Glu Leu Leu Asp Met Leu Ala Arg Ala Glu Arg Glu
       50                  55                  60
   Lys Lys
   65
<210> 2
   <211> 64
   <212> PRT
   <213> Sulfolobus solfataricus
<400> 2
   Met Ala Thr Val Lys Phe Lys Tyr Lys Gly Glu Glu Lys Glu Val Asp
   1               5                   10                  15
   Ile Ser Lys Ile Lys Lys Val Trp Arg Val Gly Lys Met Ile Ser Phe
               20                  25                  30
   Thr Tyr Asp Glu Gly Gly Gly Lys Thr Gly Arg Gly Ala Val Ser Glu
           35                  40                  45
   Lys Asp Ala Pro Lys Glu Leu Leu Gln Met Leu Glu Lys Gln Lys Lys
       50                  55                  60
<210> 3
   <211> 65
   <212> PRT
   <213> Sulfolobus acidocaldarius
<400> 3
   Met Ala Lys Val Arg Phe Lys Tyr Lys Gly Glu Glu Lys Glu Val Asp
   1               5                   10                  15
   Thr Ser Lys Ile Lys Lys Val Trp Arg Val Gly Lys Met Val Ser Phe
               20                  25                  30
   Thr Tyr Asp Asp Asn Gly Lys Thr Gly Arg Gly Ala Val Ser Glu Lys
           35                  40                  45
   Asp Ala Pro Lys Glu Leu Met Asp Met Leu Ala Arg Ala Glu Lys Lys
       50                  55                  60
   Lys
   65
<210> 4
   <211> 64
   <212> PRT
   <213> Sulfolobus shibatae
<400> 4
   Met Val Thr Val Lys Phe Lys Tyr Lys Gly Glu Glu Lys Glu Val Asp
   1               5                   10                  15
   Thr Ser Lys Ile Lys Lys Val Trp Arg Val Gly Lys Met Ile Ser Phe
               20                  25                  30
   Thr Tyr Asp Glu Gly Gly Gly Lys Thr Gly Arg Gly Ala Val Ser Glu
           35                  40                  45
   Lys Asp Ala Pro Lys Glu Leu Leu Gln Met Leu Glu Lys Gln Lys Lys
       50                  55                  60
<210> 5
   <211> 64
   <212> PRT
   <213> Sulfolobus shibatae
<400> 5
   Met Ala Thr Val Lys Phe Lys Tyr Lys Gly Glu Glu Lys Gln Val Asp
   1               5                   10                  15
   Ile Ser Lys Ile Lys Lys Val Trp Arg Val Gly Lys Met Ile Ser Phe
               20                  25                  30
   Thr Tyr Asp Glu Gly Gly Gly Lys Thr Gly Arg Gly Ala Val Ser Glu
           35                  40                  45
   Lys Asp Ala Pro Lys Glu Leu Leu Gln Met Leu Glu Lys Gln Lys Lys
       50                  55                  60
<210> 6
   <211> 64
   <212> PRT
   <213> Sulfolobus sp.
<400> 6
   Met Val Thr Val Lys Phe Lys Tyr Lys Gly Glu Glu Lys Glu Val Asp
   1               5                   10                  15
   Ile Ser Lys Ile Lys Lys Val Trp Arg Val Gly Lys Met Ile Ser Phe
               20                  25                  30
   Thr Tyr Asp Asp Asn Gly Lys Thr Gly Arg Gly Ala Val Ser Glu Lys
           35                  40                  45
   Asp Ala Pro Lys Glu Leu Leu Gln Met Leu Glu Lys Ser Gly Lys Lys
       50                  55                  60
<210> 7
   <211> 64
   <212> PRT
   <213> Sulfolobus islandicus
<400> 7
   Met Val Thr Val Lys Phe Lys Tyr Lys Gly Glu Glu Lys Gln Val Asp
   1               5                   10                  15
   Thr Ser Lys Ile Lys Lys Val Trp Arg Val Gly Lys Met Ile Ser Phe
               20                  25                  30
   Thr Tyr Asp Glu Gly Gly Gly Lys Thr Gly Arg Gly Ala Val Ser Glu
           35                  40                  45
   Lys Asp Ala Pro Lys Glu Leu Leu Gln Met Leu Glu Lys Gln Lys Lys
       50                  55                  60
<210> 8
   <211> 60
   <212> PRT
   <213> Artificial
<220>
   <223> Séquence consensus
<220>
   <221> VARIANT
   <222> (2)..(2)
   <223> Xaa est V, A ou T
<220>
   <221> VARIANT
   <222> (3)..(3)
   <223> Xaa est T ou K
<220>
   <221> VARIANT
   <222> (5)..(5)
   <223> Xaa est R ou K
<220>
   <221> VARIANT
   <222> (14)..(14)
   <223> Xaa est E ou Q
<220>
   <221> VARIANT
   <222> (17)..(17)
   <223> Xaa est T ou I
<220>
   <221> VARIANT
   <222> (30)..(30)
   <223> Xaa est A, V ou I
<220>
   <221> VARIANT
   <222> (36)..(38)
   <223> Xaa Xaa Xaa est EGG ou DN-
<220>
   <221> VARIANT
   <222> (56)..(56)
   <223> Xaa est M ou L
<220>
   <221> VARIANT
   <222> (57)..(57)
   <223> Xaa est Q ou D
<220>
   <221> VARIANT
   <222> (60)..(60)
   <223> Xaa représente A, ARAE, EKQKK, EKSGKK, ARAEREKK, ARAEKKK, ACAEREKK ou ACAEKKK
<400> 8
   Met Xaa Xaa Val Xaa Phe Lys Tyr Lys Gly Glu Glu Lys Xaa Val Asp
   1               5                   10                  15
   Xaa Ser Lys Ile Lys Lys Val Trp Arg Val Gly Lys Met Xaa Ser Phe
               20                  25                  30
   Thr Tyr Asp Asp Asn Xaa Gly Lys Thr Gly Arg Gly Ala Val Ser Glu
           35                  40                  45
   Lys Asp Ala Pro Lys Glu Leu Xaa Xaa Met Leu Xaa
       50                  55                  60